# EUROPEAN PATENT SPECIFICATION

(11) **EP 3 657 166 B1**
(45) Date of publication and mention of the grant of the patent: **18.08.2021**
(21) Application number: 18792412.1
(22) Date of filing: 20.07.2018
(51) Int. Cl.: G01N 33/03, B06B 1/02, A23L 5/30, A23L 33/115

(54) **DEVICE FOR THE CONTINUOUS UNIFORM CONDITIONING OF GROUND OLIVE PASTE USING POWER ULTRASOUND**
VORRICHTUNG ZUR KONTINUIERLICHEN GLEICHMÄSSIGEN KONDITIONIERUNG VON GEMAHLENEN OLIVENPASTEN MITTELS LEISTUNGSULTRASCHALL
DISPOSITIF DE CONDITIONNEMENT EN CONTINU ET UNIFORME DE MASSE D'OLIVES BROYÉES AU MOYEN D'ULTRASONS DE PUISSANCE

(30) Priority: 21.07.2017 ES 201730870 U
(43) Date of publication of application: 27.05.2020
(73) Proprietor: Instituto De Investigación Y Formación Agraria Y Pesquera (IFAPA). Consejeria de Agricultura, Pesca Y Desarrollo Rural. Junta de Andalucia, 41012 Sevilla (ES)
(72) Inventor: JIMÉNEZ MÁRQUEZ, Antonio, 23620 Mengíbar (Jaén) (ES); BELTRÁN MAZA, Gabriel, 23620 Mengíbar (Jaén) (ES); BEJAOUI, Mohamed Aymen, 23620 Mengíbar (Jaén) (ES)
(74) Representative: Pons
(86) International application number: PCT/ES2018/070517
(87) International publication number: WO 2019/016427

(56) References cited:
- ES-A1- 2 206 015
- ES-A1- 2 327 308
- JULIO BELTRÁN ORTEGA ET AL: "Novel technologies for monitoring the in-line quality of virgin olive oil during manufacturing and storage : Monitoring the quality of olive oil", JOURNAL OF THE SCIENCE OF FOOD AND AGRICULTURE, vol. 96, no. 14, 18 May 2016 (2016-05-18), pages 4644-4662, XP055548387, GB ISSN: 0022-5142, DOI: 10.1002/jsfa.7733
- Riccardo Amirante ET AL: "Ultrasound in Olive Oil Extraction" In: "Products from Olive Tree", 26 October 2016 (2016-10-26), InTech, XP055548423, ISBN: 978-953-51-2725-3 pages 43-53, DOI: 10.5772/64765, the whole document

## Description

### OBJECT OF THE INVENTION

The present invention pertains to the technical field of apparatuses especially adapted to transmit ultrasound-frequency mechanical vibrations, as well as to the field of the fatty oil production by extracting raw materials, and it particularly relates to a continuous and uniform conditioning device of ground olive mass by means of power ultrasound capable of quickly, uniformly and continuously conditioning ground olive mass during the olive oil extraction process.

### BACKGROUND OF THE INVENTION

The current methods of producing virgin olive oil comprise a number of successive steps, including the steps of grinding and mixing; next, a liquid-solid separation is carried out by means of centrifugation where the oil of the solid material and of the vegetation water is separated, concluding the process with a liquid-liquid separation that enables the oil to be clarified in order to eliminate residual solid material and water before the storage thereof.

The mixing step of the olive paste is that which has a greater impact on the yield of extraction and the quality and composition of the oils obtained due to the set of chemical and biochemical reactions that are developed in this operation. This step is carried out in thermo-mixers which consist of cylindrical or semi-cylindrical tanks with a heating jacket, equipped with a vertical or horizontal shaft. This shaft has a plurality of blades that enable the olive paste to be stirred. The main objective thereof is to homogenise the mass and thermally treat it to thus facilitate the coalescence of the oil droplets and the formation of a continuous oily phase.

The two fundamental variables of this mixing step are time and the temperature of the mass, which greatly define the effectiveness of the following treatments and the characteristics of the oil obtained. It is known that in order to extract the greatest amount of oil possible it is necessary to apply a high temperature during this process during a prolonged period of time. However, this harms the quality of the oil obtained, due to the fact that the oxidative processes of the oil and the aeration of the mass are accelerated, leading to a deterioration of the organoleptic characteristics.

One of the reasons that make it necessary to extend the mixing time is the time that the olive mass takes to reach the objective temperature. Normally, in the current mixers the heating of the mass is carried out by means of heat transfer from the surface of a heating jacket to the inside of the paste. This transfer is carried out slowly, as a result of the low heat capacity of the olive mass, and furthermore, a very high temperature should not be applied in order to avoid overheating as a result of thermal inertia.

The use of ultrasound has been recently proposed to continuously pre-heat the olive mass prior to subsequent mixing, which enables the pre-heating time in the current mixers to be eliminated. Applying this technology to the ground olive mass leads to a series of phenomena in the medium, similar to those that take place during mixing, such as micro-stirring, the sponge effect or the transfer of an amount of movement, energy and matter.

Prior works on the treatment of ground olives by power ultrasound showed that the characteristics of the olives used (variety, degree of ripeness, moisture content of the olives, temperatures of the olives, etc.) determine the frequency and power of the ultrasound that has to be applied to obtain efficient treatment.

A known device for heating a mass of ground olives is given in ES2327308 which comprises an ultrasound device to heat the olive mass, an ultrasound applicator, first and second temperature sensors and a controller that acts on the ultrasound applicator to modify the frequency and the power of the applied ultrasound to the olive mass based on the outputs of the first and second temperature sensors.

Conditioning devices of olives masses based on a multifrequency application of power ultrasound, prior to the injection thereof in a horizontal centrifugal decanter for extracting oil, and without using a mixer, but rather quick pre-heating systems of the mass before the mixing thereof, all based on water heat exchangers, are not known in the current state of the art.

### DESCRIPTION OF THE INVENTION

The object of the invention consists of a device for continuously conditioning a ground olive mass by applying multifrequency power ultrasound. The low frequency or power ultrasound are acoustic waves with frequencies between 16 - 100 kHz that produce permanent effects on the medium in which it is applied and with intensities that can vary from 0.1 W/cm² to several kW/cm².

To do so, the device comprises a multifrequency element for applying ultrasound, an element for regulating the frequency and the power of the ultrasound applied, temperature sensors, sensors for characterising the olive paste and flow meters.

According to a first preferred embodiment of the invention, the device for conditioning the ground olive mass comprises:
- a sensor for determining the physical and chemical characteristics of an input flow of ground olive mass,
- a first temperature sensor of the ground olive mass,
- a sensor and controller of the input flow of the ground olive mass,
- an element for applying power ultrasound to the flow of ground olive,
- a second temperature sensor of the output flow of ultrasound-treated -olive mass,
- a receiving container of the output flow of ground and treated olive mass, also referred to as a mass receptacle, and
- an element for controlling the frequency and power of the power ultrasound applied and of the flow of ground olive mass, based on the information provided by the temperature sensors, the flow sensor and the sensors of the physical and chemical characteristics of the ground olive mass, of the pomace and of the oil.

Subsequent to the conditioning of the mass, the oil of the mass is extracted, for which reason it has an oil extractor, a sensor for determining the physical and chemical characteristics of the pomace byproduct and a sensor for determining the physical and chemical characteristics of the oil product.

The element for applying power ultrasound can be any of those that enable power ultrasound to be applied to a continuous flow of ground olive mass, being able to have a geometry such that it facilitates the coupling thereof to a duct through which the mass circulates, as well as to apply the power ultrasound directly to the ground olive mass via the pipe through which said mass flows. Furthermore, the device enables the cooling thereof by means of air or any fluid or inert gas.

According to another more preferred embodiment of the invention, the device for applying power ultrasound incorporates piezoelectric transducers coupled to the pipe through which the ground olive mass circulates, on which the device for controlling the power ultrasound acts, which enables both the power and the frequency of the power ultrasound applied to be controlled. This enables the temperature of the ground mass that passes through the tubes to increased, from low temperatures of approximately 5-10°C to working temperatures of 15-60°C.

In the present document, it is understood that the control of applying power ultrasound includes a control of power between 150 W and 10 kW and a control of frequency between 20 and 100 kHz.

The device for controlling the ultrasound applied can be any device that to which the necessary logic can be provided, and which can modify the characteristics of the ultrasound applied and of the mass input flow. To measure the physical and chemical characteristics of the ground olive mass at the inlet of the device for applying ultrasound, of the pomace and of the oil, non-invasive and non-destructive sensors are used which enable the information needed to be obtained in real time and which can be coupled to the pipes of the process and, preferably, are based on the emission of infrared radiation.

On the other hand, to determine and control the flow of olive mass in the device for applying power ultrasound any mass sensor coupled to a mass impeller pump of adjustable flow by means of an external device can be used.

Among the multiple advantages provided by the conditioning device described, it is first worth noting those referring to lower energy consumption and low CO₂ emissions, since the ultrasound is responsible for directly heating the mass, while the other products need heating apart from the heating water, with the subsequent fuel consumption and CO₂ emission by the boilers existing in the mills.

Likewise, the extractability of the oil is improved, without organoleptic alterations being produced. The control of the heating of the olive mass is also improved by having a plurality of control elements for optimising flows, time and frequency of ultrasound to be applied, and the capacity for activating or deactivating certain enzyme producers of volatile compounds increases thanks to the ultrasonic frequency used, which enables modulation of the sensory attributes of the oil produced. Finally, by being able to eliminate the mixing step of the mass, the versatility of the mills is improved by increasing the available space.

A first process of heating the ground olive mass that uses the device described above comprises the following steps:
1) Measurement of the input temperature of the ground olive mass in the device, by means of the first temperature sensor,
2) Application of power ultrasound to the ground olive mass that passes through the device for applying power ultrasound; according to the preferred embodiment of the invention, the power ultrasound applied would be between 120 W and 12000 W, and the frequency thereof between 20 kHz and 100 kHz, and
3) Measurement of the output temperature of the ground olive mass of the device for applying power ultrasound.

The controller acts based on the input and output temperatures of the ground olive mass, of the frequency and power of the power ultrasound applied, and of the input flow to the device for applying the power ultrasound. According to the embodiment herein described, the heating of the ground olive mass will be produced from low temperatures, approximately 5-10°C, to temperatures of 15-60°C, while the inlet flow of ground olive mass to the device could be comprised between 50 kg/h and 30000 kg/h.

A second process of heating the ground olive mass by means of the device described comprises the following steps:
1) Physical and chemical characterisation by means of a non-invasive sensor of the input olive mass,
2) Measurement of the input temperature of the ground olive mass,
3) Application of power ultrasound to the ground olive mass to a power between 120-1200 W and a frequency of 20-100 kHz,
4) Measurement of the output temperature (T2) of the olive mass,
5) Storage of the ground and treated olive mass with the device for applying the power ultrasound in a stainless steel tank under conditions that are isolated from the outer conditions, preferably, isolated from the environment,
6) Transfer of the mass to an oil extracting device,
7) Physical and chemical characterisation by means of non-invasive sensors of the ground and extracted olive mass at the output of the extraction system,
8) Physical and chemical characterisation by means of non-invasive sensors of the extracted oil at the output of the extraction system, and
9) Control of the frequency and power of the power ultrasound applied, to obtain an outlet temperature of the device of the desired ground olive mass, a degree of extractabiliity and desired characteristics of the oil.

This control is carried out by means of a SCADA-type control system, based on the information coming from the different temperature sensors and the physical and chemical characterisation of the olive mass, of the ground and extracted olive mass and of the extracted oil; of the input flow of the ground olive mass to the device for applying the power ultrasound, and of the feed flow of the system for extracting the ground and treated olive mass.

### DESCRIPTION OF THE DRAWINGS

In order to complement the description being made and with the object of helping to better understand the characteristics of the invention, in accordance with a preferred practical exemplary embodiment thereof, said description is accompanied, as an integral part thereof, by a set of drawings where, in an illustrative and non-limiting manner, the following has been represented:

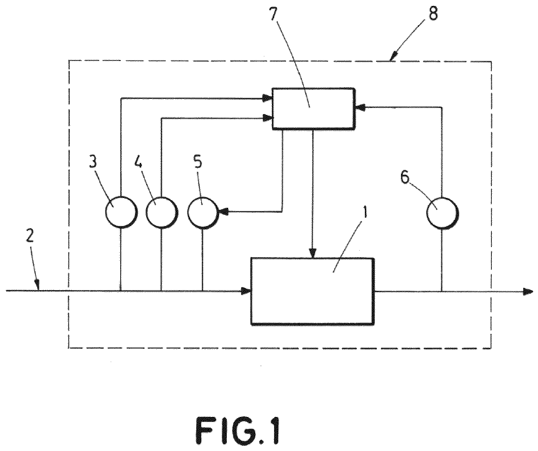

Figure 1 shows a diagram of the continuous and uniform conditioning device of ground olive mass by means of power ultrasound.

### PREFERRED EMBODIMENT OF THE INVENTION

With the help of the previously mentioned figures, a detailed explanation of a preferred exemplary embodiment of the object of the present invention is provided below.

The continuous and uniform conditioning device of ground olive mass by means of power ultrasound that is described is made up of a multifrequency ultrasound applicator (1), which acts on a mass of ground olive paste circulating through the inside of a stainless steel duct (2) to increase the temperature thereof.

In a position in front of the input of the paste flow in the ultrasound applicator (1), there is a first temperature sensor (3), a physical and chemical sensor (4) and a flow meter (5), while a second temperature sensor (6) is arranged in a position behind the ultrasound applicator (1).

The first temperature sensor (3) determines the temperature of said flow before applying ultrasound, while the second temperature sensor (6) measures it after the mass has been heated by means of the ultrasound applied.

It is envisaged that the physical and chemical sensor (4), which can be coupled to the duct (2), is of a non-invasive and non-destructive type and is based on the emission of infrared radiation to obtain the information needed in real time, related to parameters of the olive paste such as moisture, fat content, degree of ripeness, extractability and other rheological and compositional characteristics. On the other hand, to determine and control the flow of olive mass, a mass sensor, coupled to a mass impeller pump of adjustable flow is used as a flow meter (5).

The ultrasound applicator (1) has a geometry such that it facilitates the coupling thereof to a duct (2) through which the mass circulates, which is intended to be heated. It can also apply power ultrasound directly to the continuous flow of ground olive mass or indirectly via the duct (2) through which said mass flows. Furthermore, the ultrasound applicator (1) enables the cooling thereof by means of air or other inert fluid.

According to the preferred embodiment herein described, the device for applying ultrasound (1) incorporates piezoelectric transducers coupled to the duct (2), on which a controller (7) acts, to modify both the power and the frequency of the power ultrasound applied to increase the temperature of the mass based on the data provided by the first (3) and second (6) temperature sensors and the physical and chemical sensor (4). The controller (7) also acts on the mass impeller pump of an adjustable flow of the flow meter (5) to thus regulate the circulating flow of olive paste at the input of the device for applying ultrasound (1).

Furthermore, the incorporation of a containing casing (8) is envisaged, having means for coupling to the duct (2), the casing (8) which houses the ultrasound applicator (1), the first (3) and second (6) temperature sensors, the physical and chemical sensor (4), the flow meter (5) and the controller (7).

The process for extracting oil that uses the device described begins with the characterisation of the characteristics of the ground olive mass by means of the physical and chemical sensor (4), followed by measuring the temperature of the mass by means of the first temperature sensor (3). Based on both measurements, the controller (7) acts on the flow meter (5) to establish the flow of olive mass to be treated by the device.

Next, the ground olive mass passes through the power ultrasound applicator (1) for the conditioning thereof, wherein the frequency and power of the power ultrasound applied are regulated by means of the controller (7) with the information provided by the first temperature sensor (1) and the physical and chemical sensor (4).

Subsequently, the temperature of the continuous flow of ground olive mass already treated by the ultrasound applicator (1) is measured a second time. In the case that said temperature is not the desired temperature, the controller (7) can simultaneously modify both the frequency and the power of the ultrasound applied, such as the flow of ground olive mass.

Finally, the ground and treated olive mass is recovered in a mass receptacle, where it is stored before being sent by means of a pump to a system for extracting olive oil, or it is subjected to mixing in the mass receptacle during a specific amount of time before being sent for extraction.

## Claims

1. A continuous and uniform conditioning device of ground olive mass by means of power ultrasound, intended to heat an olive mass resulting from milling and circulating through the inside of a duct (2), the device comprising:
- an ultrasound applicator (1) for applying power ultrasound to the olive mass,
- a first temperature sensor (3) for determining the temperature of the olive mass at a time immediately prior to applying ultrasound,
- a physical and chemical sensor (4) for determining physical and chemical parameters of the olive mass prior to applying ultrasound,
- a flow meter (5) for regulating the flow of olive mass circulating through the duct (2),
- a second temperature sensor (6) for determining the temperature of the olive mass at a time immediately after applying ultrasound, and
- a controller (7) that acts on:
- the ultrasound applicator (1) to modify the frequency and the power of the power ultrasound applied to the olive mass, and
- the flow meter (5) for regulating the flow of olive mass circulating through the duct (2),
based on the determinations obtained by the first (3) and the second (6) temperature sensor and the physical and chemical sensor.

2. The conditioning device according to claim 1, **characterised in that** the ultrasound applicator (1) incorporates piezoelectric transducers for modifying the frequency and the power of the ultrasound applied to the olive mass.

3. The conditioning device according to any of the preceding claims, **characterised in that** it incorporates a protective casing (8) having means for coupling to the duct (2).

## Patentansprüche

1. Kontinuierliche und gleichmäßige Aufbereitungsvorrichtung für gemahlene Olivenmasse mittels Leistungsultraschall, die dazu bestimmt ist, eine Olivenmasse zu erwärmen, die aus dem Mahlen und Zirkulieren durch das Innere eines Kanals (2) stammt, wobei die Vorrichtung umfasst:
- einen Ultraschallapplikator (1) zum Anlegen von Leistungsultraschall an die Olivenmasse,
- einen ersten Temperatursensor (3) zum Bestimmen der Temperatur der Olivenmasse zu einem Zeitpunkt unmittelbar vor dem Anlegen von Ultraschall,
- einen physischen und chemischen Sensor (4) zum Bestimmen der physischen und chemischen Parameter der Olivenmasse vor dem Anlegen von Ultraschall,
- einen Durchflussmesser (5) zum Regeln des Durchflusses von Oivenmasse, die durch den Kanal (2) zirkuliert,
- einen zweiten Temperatursensor (6) zum Bestimmen der Temperatur der Olivenmasse zu einem Zeitpunkt unmittelbar nach dem Anlegen von Ultraschall und
- eine Steuerung (7), die auf Folgendes einwirkt:
- den Ultraschall-Applikator (1) zum Modifizieren der Frequenz und der Leistung des Leistungsultraschalls, der an die Olivenmasse angelegt wird, und
- den Durchflussmesser (5) zum Regeln des Durchflusses von Oivenmasse, die durch den Kanal (2) zirkuliert,
Basierend auf Bestimmungen, die durch den ersten (3) und den zweiten (6) Temperatursensor und den physischen und chemischen Sensor erhalten werden.

2. Aufbereitungsvorrichtung nach Anspruch 1, **dadurch gekennzeichnet, dass** der Ultraschall-Applikator (1) piezoelektrische Wandler zum Modifizieren der Frequenz und der Leistung von Ultraschall, der an die Olivenmasse angelegt wird, beinhaltet.

3. Aufbereitungsvorrichtung nach einem der vorstehenden Ansprüche, **dadurch gekennzeichnet, dass** diese ein Schutzgehäuse (8) integriert, das Mittel zum Koppeln mit dem Kanal (2) aufweist.

## Revendications

1. Dispositif de conditionnement continu et uniforme de masse d'olives broyées au moyen d'ultrasons de puissance, destiné à chauffer une masse d'olives issue du broyage et circulant à travers l'intérieur d'un conduit (2), le dispositif comprenant :
- un applicateur à ultrasons (1) pour appliquer des ultrasons de puissance à la masse d'olives,
- un premier capteur de température (3) pour déterminer la température de la masse d'olives à un instant immédiatement avant l'application d'ultrasons,
- un capteur physique et chimique (4) pour déterminer les paramètres physiques et chimiques de la masse d'olives avant l'application d'ultrasons,
- un débitmètre (5) pour réguler le débit de masse d'olives circulant à travers le conduit (2),
- un deuxième capteur de température (6) pour déterminer la température de la masse d'olives à un instant immédiatement après l'application d'ultrasons, et
- un contrôleur (7) qui agit sur :
- l'applicateur d'ultrasons (1) pour modifier la fréquence et la puissance des ultrasons de puissance appliqués à la masse d'olives, et
- le débitmètre (5) pour réguler le débit de masse d'olives circulant à travers le conduit (2),
en fonction des déterminations obtenues par le premier (3) et le deuxième (6) capteur de température et le capteur physique et chimique.

2. Dispositif de conditionnement selon la revendication 1, **caractérisé en ce que** l'applicateur d'ultrasons (1) incorpore des transducteurs piézoélectriques pour modifier la fréquence et la puissance des ultrasons appliqués à la masse d'olives.

3. Dispositif de conditionnement selon l'une quelconque des revendications précédentes, **caractérisé en ce qu'**il incorpore une enveloppe de protection (8) ayant des moyens de couplage au conduit (2).
